# EUROPEAN PATENT SPECIFICATION

(11) **EP 0 560 937 B1**
(45) Date of publication and mention of the grant of the patent: **24.03.1999**
(21) Application number: 92903167.2
(22) Date of filing: 04.12.1991
(51) Int. Cl.: A61K 9/54, A61K 31/415

(54) **PHARMACEUTICAL COMPOSITIONS**
PHARMAZEUTISCHE ZUSAMMENSETZUNGEN
COMPOSITIONS PHARMACEUTIQUES

(30) Priority: 05.12.1990 US 622875
(43) Date of publication of application: 22.09.1993
(73) Proprietor: SMITHKLINE BEECHAM CORPORATION, Philadelphia Pennsylvania 19101 (US)
(72) Inventor: MARSHALL, Keith, Doylestown, PA 18901 (US); THIELE, William, Jay, London SW7 1NL (GB)
(74) Representative: Thompson, Clive Beresford
(86) International application number: US9109221
(87) International publication number: WO9210173

(56) References cited:
- EP-A- 202 051
- EP-A- 237 200
- EP-A- 290 229
- EP-A- 393 747
- EP-A- 431 877
- WO-A-88/03795
- US-A- 4 940 588

## Description

### FIELD OF THE INVENTION

This invention relates to solid pharmaceutical compositions containing cimetidine.

### BACKGROUND OF THE INVENTION

Cimetidine is a histamine H₂-antagonist which has been described in U.K. Patent Specification 1,397,436. Cimetidine has been shown to be useful in the treatment of duodenal, gastric, recurrent and stomal ulceration, and reflux oesophagitis and in the management of patients who are at high risk from haemorrhage of the upper gastrointestinal tract.

Cimetidine has been made available to patients in a variety of dosage forms; for example, tablets, granules, syrups and suspensions. In most, if not all, of these dosage forms, the cimetidine is in an immediate-release form; that is to say the nature of the formulation is such that by the time the cimetidine leaves the stomach, it is either in solution or is in the form of a suspension of fine particles, i.e. a form from which it can be readily absorbed.

Coating agents which prevent release of an active ingredient in the stomach but which allow release in the intestines are known as enteric coating agents and many such substances are known in the art for this purpose. Similarly, agents which form a matrix in which the active ingredient is embedded are known to modify the release of the active ingredient. However, it has been found that, when many such release delaying substances are used in conjunction with cimetidine, although release is delayed, the bioavailability of the cimetidine is substantially reduced.

EP-A-0 431 877 (a document representing prior art according to Art 54(3) EPC) describes an enteric coated oral dosage form of cimetidine. EP-A-0 290-299 describes pharmaceutical granules comprising cimetidine and a co-polymer of dimethylamino ethyl acrylate and neutral methacrylic acid esters. EP-A-0 393 747 describes a pharmaceutical composition designed to transport mebeverine to the lumen of the lower GI tract. EP-A-0 202 051 describes a composition presented as a gelatin capsule and containing three different types of spheroids which have different coatings, to provide different release characteristics.

### SUMMARY OF THE INVENTION

The present invention provides a pharmaceutical composition comprising an H₂ antagonist characterised in that the H₂ antagonist is present in a modified-release phase comprising a polymer matrix which is selected from the group consisting of a non-ionic neutral copolymer of ethyl acrylate and methyl acrylate, acrylic and methacrylic acid esters, ethyl cellulose, hydroxypropyl methylcellulose, gelatin, waxes or mixtures thereof; the polymer matrix material being present in an amount of 10 to 20% (w/w) of polymer relative to H₂ antagonist.

The composition comprises a plurality of H₂ antagonist in matrix cores which have different release rate phases. The different release rates are provided for by matrix cores having a modified-release of the active ingredient, over time. The modified-release phase may contain two, three, four or more phases of modified-release polymer matrix-cores (or matrixes). The composition preferably comprises an immediate-release phase of cimetidine as well as the modified release phase. The modified release phase alone or in combination with immediate release of cimetidine is able to extend the duration of action of cimetidine and thereby provide improved bioavailability of cimetidine.

### DETAILED DESCRIPTION OF THE INVENTION

In one aspect, the present invention is a pharmaceutical composition which comprises an H₂ antagonist in a polymer matrix core. The H₂ antagonist polymer matrix is a composition comprising the H₂ antagonist in a polymer matrix. The modified release phase, preferably composed of pellets, comprises a plurality of discrete polymer matrix particules suitable for combination into a pharmaceutical composition. The composition may comprise multiple release phases of matrixes each of which comprises the H₂ antagonist incorporated, independently, into a polymer matrix.

The modified release phase matrix-core may be un-coated (hereinafter referred to as the "uncoated matrix") or coated (hereinafter referred to as a "coated matrix") with a release-delaying substance. Preferably, the release delaying substance is present in an amount of from 2 to 30% (w/w) relative to the matrix-core. More preferably, the release-delaying substance is present in an amount of from 5 to 25% (w/w).

Another aspect of the present invention provides for various combinations of modified-phase release pellets such that uncoated matrix pellets may be in combination with immediate release phase pellets of the H₂ antagonist, preferably cimetidine. Another combination is the coated matrix pellets in combination with immediate release phase pellets of cimetidine, as well as the uncoated and coated matrix pellets in combination with each other. The matrix-core pellets may be coated independently with different release-delaying substances all of which may be combined with the uncoated or immediate release phase pellets of cimetidine.

Yet another aspect of the present invention provides for an additional modified-release phase to be present in combination with the coated or un-coated matrix, which additional modified-release phase comprises at least one core of cimetidine coated with a release-delaying substance (hereinafter referred to as "immediate coated").

The release-delaying substance is present in an amount of from 2 to 30% (w/w) relative to the core. Preferably the substance is present in an amount of 5 to 25% (w/w).

The invention further provides for combinations of the immediate coated pellets with coated matrix and immediate release pellets of cimetidine, as well as combinations of immediate coated pellets with uncoated matrix and immediate release pellets, as well as immediate coated pellets with coated matrix and un-coated matrix pellets. The combination of all four pellets (immediate-release, immediate coated, un-coated matrix and coated matrix) is also contemplated as a further aspect of the present invention and is described below in its preferred embodiment :
a) an immediate-release phase of cimetidine;
b) a "first" modified release phase which comprises a matrix-core which comprises cimetidine incorporated into a polymer matrix;
c) a "second" modified release phase matrix-core which comprises cimetidine incorporated into a polymer matrix coated with a first modified release-delaying substance in an amount of from 2 to 30% (w/w) relative to the matrix-core (hereinafter referred to as a "coated matrix"); and
d) a "third" modified-release phase which comprises at least one core of cimetidine coated with a second modified release-delaying substance in an amount of from 2 to 30% (w/w) relative to the core (hereinafter referred to as "immediate coated").

As the release-delaying substance can independently vary with the matrix core or the immediate release pellets of cimetidine all combinations of such varying coatings are contemplated within the scope of this invention. Further, as the polymer matrix can vary with the release phase desired, all combinations of varying polymer matrixes are also contemplated within the scope of this invention.

Examples of H₂ antagonists useful in the present invention include, but are not limited to, cimetidine, ranitidine, famotidine, nizatidine and roxatidine.

Immediate release phase, as used herein, is intended to mean a short pulse, i.e., a dissolution time and absorption from the gastric juices from immediate release to about 45 minutes. The immediate-release phase of the present invention contributes to a first pulse of cimetidine in the stomach. Such immediate-release formulations for instance of cimetidine are old and well known to those skilled in the art, for example, U.S. Patent No. 4,024,271 issued May 17, 1977. The immediate release phase pellets of the present invention, as described in the Example Section, herein meet such criteria. The immediate release phase of the present invention preferably comprises cimetidine as a pellet or granule (one core) not comprising a delayed released susbstance.

Modified release phase, as used herein, is intended to mean a controlled release of cimetidine such that cimetidine is not immediately and completely released into the stomach, within the time frame noted above, and remains available to the mammal for release over a prolonged period of time. The release may occur in the stomach or the intestinal tract.

The immediate coated release pellets of the present invention, are made from coated "immediate release" pellets of cimetidine. However it is within the scope of this invention that any immediate release form of cimetidine is contemplated and not limited to a bead or pellet formation as described herein. Bulk cimetidine, granulated cimetidine and coated cimetidine particles thereof are but one aspect of the present invention. The enteric coating art is well known to those skilled in the art and is described in Example 1 herein. The immediate coated release pellets of the present invention will allow for release of the cimetidine over a period of time in the intestinal tract (dependent upon the type and amount of coating chosen).

The "release delaying substance" of the present invention, as used herein, is coating agent which protects the active ingredient, the H₂ antagonist from immediate degredation in the stomach.

The modified release phase of an uncoated matrix pellet, as used herein, will result in a pulse of cimetidine which, depending upon the polymer used in the matrix itself, continue to release cimetidine for a period of time exceeding that of the immediate release. Preferably, the release will continue for up to 8 hours.

The modified release phase of a coated matrix pellet as used herein, results in the absence of available cimetidine in the stomach and allows for its release in the intestinal tract , i.e. as a "third" pulse in combination with immediate and uncoated matrix pellets. Dependent upon the coating and polymer used in the matrix a prolonged release of cimetidine in the intestinal tract will occur over an extended period of time. In this manner the duration of action of cimetidine can be extended providing good bioavailability.

Extending the duration of action of cimetidine increases the rate of healing in gastric or duodenal ulceration and is advantageous in disease states such as gastroesophageal reflux disease, dyspepsia or stress ulceration where prolonged control of acid secretion is desirable.

The cimetidine may be present as the free base or as a pharmaceutically acceptable salt, for example the hydrochloride salt.

The immediate-release phase of cimetidine can exist in any of the commonly used types of solid pharmaceutical composition, for example, as tablets, pellets or granules which can optionally be coated with a coating agent which dissolves in the gastric juices or which can optionally be contained within a gelatin capsule.

The modified release phase matrix can similarly exist as a tablet, pellet or granule and is optionally coated with the release-delaying substance. The additional modified-release phases, such as the immediate coated may also exist as a tablet, pellet or granule coated with the same, or different release-delaying substance.

The immediate and modified-release phases can be presented separately or more conveniently combined in a single dosage form. Thus, for example, a combination can take the form of immediate-release phase pellets, "first modified-release phase" un-coated matrix pellets, "second modified-release phase" coated matrix pellets, and "third modified-release phase" immediate coated pellets, all contained within a gelatin capsule. It is possible to have various combinations, such as immediate with coated matrix, immediate with uncoated matrix, immediate with immediate coated and one of the two matrix pellets, etc.

The present invention can further comprise combinations of the above described phases with yet another phase, that of an immediate-release phase of cimetidine which can be coated with a coating agent which dissolves in the gastric juices or which can optionally be contained within a gelatin capsule. Such coating agents are well known to those skilled in the art and include, but are not limited to hydroxypropyl methylcellulose, or methyl cellulose.

The immediate release phase and the modified release phase (uncoated matrix, coated matrix and immediate coated) are preferably contained in a single dosage form. The immediate release phase and modified release phase are preferably composed of pellets or beads (used interchangeably herein). The pellets are preferably contained in a capsule, which is preferably made of gelatin.

For particulate dosage forms such as pellets or granules and in a two phase system, of the total amount, preferably the immediate release phase is present in an amount of about 5 to 40% (w/w). The first and second modified release substance (the coated or un-coated matrix respectively) is thereby present in an amount of 60 to 95% (w/w) relative to the immediate release dosage form. Preferably, either matrix is present in an amount of 70 to 90% (w/w) relative to the immediate release form of 10 to 30% (w/w).

For a three phase system, including both the coated and un-coated matrix, of the total amount, the immediate release is present in an amount of about 5 to 40%, the coated matrix in an amount of about 10 to 85% (w/w) relative to the immediate release dosage and the immediate coated substance is present in an amount of about 10 to 85% (w/w) also relative to the immediate release dosage form. Preferably the immediate release is about 5 to 30%, the coated matrix about 20 to 75% (w/w), and the immediate coated is also about 20 to 75% (w/w). The modified release substances should preferably be present in about 66 to 75% total (w/w) of the cimetidine dosage form.

For the four phase system, the immediate release is present in an amount of about 5 to 40%, the (un)-coated matrix is present in an amount of about 10 to 75% (w/w) relative to the immediate release dosage and the immediate coated substance is present in an amount of about 5 to 75% (w/w) also relative to the immediate release substance.

Preferably the immediate release is present in an amount of about 5 to 30%, the coated matrix in an amount about 20 to 70% (w/w), the un-coated matrix in an amount about 20 to 70% (w/w), and the immediate coated substance is present in an amount about 10 to 30% (w/w). As in the case of the three phase system, the modified release substances should preferably be present in about 66 to 75% total (w/w) of the total dosage form.

Suitably the coating agents used for the second and/or third modified-release substance is an enteric coating agent selected from copolymers based on methacrylic acid and ethyl acrylate, copolymers based on methacyrlic acid and methacrylates (also referred to as methacrylic acid copolymers, Type A-C United States Pharmacopia, 22nd Edition), copolymers based on hydroxypropyl methylcellulose phthalate, cellulose acetate phthalate, polyvinyl acetate phthalate, or mixtures thereof.

Preferably the coating agent is Eudragit ™L30D which is an aqueous dispersion containing 30% (w/w) of an acrylic resin formed from a copolymer based on polymethacrylic acid and acrylic acid esters. The acrylic resin is soluble in intestinal juice from pH 5.5 upwards.

The matrix material comprising the modified release phase contains a suitable polymer which forms a matrix from which the cimetidine can be gradually released. Examples of suitable polymers include non-ionic neutral copolymer based on ethyl acrylate and methyl acrylate (also referred to as polyacrylates), acrylic and methacrylic acid esters, ethyl cellulose, hydroxypropyl methylcellulose, gelatin or various waxes (such as white, carnauba, stearyl alcohol, stearic acid, polyethylene glycol, castor wax, polyethylene glycol monostearate and triglycerides) or mixtures thereof.

Preferably the polymer is Eudragit™ NE 30D which is an aqueous dispersion containing 30% (w/w) of a neutral copolymer based on ethyl acrylate and methyl acrylate. Preferably this polymer is present in an amount of 10 to 20% (w/w) of polymer relative to the cimetidine (based upon dry weight of polymer).

The formulations of the present invention may further comprise additional excipents and agents well known in the coating art such as:
plasticisers, e.g. acetylated monoglycerides, diethyl phthalate, triacetin, citric esters such as triethyl citrate, acetyl triethyl citrate, tributyl-citrate or acetyl tributyl citrate, propylene glycol, tributyrine, butylphthalylbutyl glycolate, glycerine, polyethylene glycols, glyceryl triacetate, dibutyl sebacate, dibutyl phthalate, castor oil or acetyl monoglyceride, polysorbates and sodium lauryl sulfate;
lubricants, e.g. calcium stearate, colloidal silicon dioxide, mineral oil, magnesium stearate, polyethylene glycol or talc; or
film disintegrating agents, e.g. lactose, saccharose, starch, cellulose, kaolin, polyvinyl alcohol or hydroxypropyl methyl cellulose.

The coating agents of the additional, or second and third modifed-release phase substances need not be the same, however, conveniently they are essentially the same.

The process of producing immediate release substance is well known to those skilled in the art. For instance the H₂ antagonist can be granulated in accordance with standard pharmaceutical techinques; thus is can be mixed with a solution of a binding agent in a conventional mixing device or it can be subjected to fluidised bed granulation methods as known in the art.

The process of producing the coated modified-release phase tablets, pellets and particules is also well known to those skilled in the art and may be readily achieved by suspending the material to be coated in fluidized bed equipment, spraying the coating solution onto the material and subsequently drying. Alternatively, the coating can be carried out in pans designed for such purpose and employing similar spraying techniques to coat materials and subsequently drying thereafter.

The immediate-release and modified-release phase forms of this invention suitably comprise other standard pharmaceutically acceptable carriers or excipients, for example starch, celluloses, sodium croscarmellose, sodium starch glycoate, crospovidone, polyvinyl alcohol, polyvinylpyrrolidione, gelatin, low viscosity hydroxypropyl methylcellulose, lactose, sucrose, talc, kaolin, colloidal silicon dioxide, magnesium stearate, or stearic acid.

The invention will now be further illustrated by means of the following examples.

### Example 1

Uncoated immediate-release cimetidine pellets, un-coated matrix pellets, and immediate coated pellets are contained within a hard gelatin capsule.

### Manufacture of Immediate-Release Pellets

| Ingredients | % w/w |
|---|---|
| Cimetidine | 85 |
| Microcrystalline Cellulose | 12 |
| Gelatin | 3 |
| Water | (qs) |

The cimetidine and part of the microcrystalline cellulose are dry blended in a high shear mixer. Mixing is continued while a solution of the gelatin in water is added. When homogeneously massed the material is passed through an extruder and recirculated through it once. The extrudate is transferred to a Marumerizer bowl and spheronized. The rest of the microcrystalline cellulose is used as dusting powder to facilitate this stage of the process. The pellets are discharged and spread out on trays to be dried in a hot air oven. The dried pellets are screened via a 1.4mm sieve to remove oversize and via a 0.6mm sieve to remove undersized fractions. Pellets fractions between 600 microns and 1400 microns in diameter are retained. These dried uncoated immediate-release pellets therefore contain 85% of cimetidine.

### Coating of Immediate-Release Pellets to yield Immediate Coated Pellets

### Composition of coating suspension % w/w

| | |
|---|---|
| Eudragit ™L30D | 51.2 |
| Triethyl citrate | 2.3 |
| Colloidal silicon dioxide | 1.2 |
| Water | 45.3 |

Pellets obtained as described above are coated by bottom spraying with the coating suspension in Fluidised Bed equipment until a 20% gain in weight is achieved. These coated pellets therefore contain 85/120 = 70.8% of cimetidine. The coated pellets are dried in situ before discharge, and then allowed to cure overnight at room temperature, while spread out on trays. The approximate weight of a coated pellet is about 0.8 mg.

### Manufacture of Cimetidine Polymer Matrix-Pellets

### (Uncoated Matrix Pellets)

| Ingredients of Core | % w/w |
|---|---|
| Cimetidine | 75 |
| Microcrystalline Cellulose | 10 |
| Gelatin | 3 |
| Eudragit NE 30 D | 12* |
| Water | (qs) |

| | |
|---|---|
| * Calculated as dry weight of polymer | |

The cimetidine polymer matrix-pellets are prepared in similar manner to the immediate-release pellets except that the Eudragit ™NE 30 D suspension is added prior to the addition of the gelatin solution. Dried uncoated cimetidine polymeric matrix-pellets therefore contain 75% of cimetidine.

### Encapsulation

The coated and uncoated pellets are prepared as in Example 1 are filled (encapsulated) into capsules, such that one capsule contains:
58.8 mg of uncoated immediate release pellets comprising 50 mg cimetidine;
70.6 mg of immediate coated pellets comprising 50mg cimetidine; and
266.7 mg of coated matrix pellets comprising 200 mg cimitidine.

Thus two capsules provides a 600mg dose of cimetidine.

### Example 2

Immediate-release cimetidine pellets, and uncoated matrix are contained within a hard gelatin capsule.

### Manufacture:

The un-coated matrix pellets and immediate release cimetidine pellets are prepared as in Example 1.

### Encapsulation:

These pellets are filled (encapsulated) into capsules, such that one capsule contains:
88.2mg of uncoated immediate release pellets comprising 75mg cimetidine, and
300mg of uncoated matrix pellets comprising 225mg cimetidine.

When prepared in accordance with these procedures, one capsule would provide a 300mg dose of cimetidine.

Alternatively, the pellets may also be encapsulated such that one capsule contains:
58.8 mg of uncoated immediate release pellets comprising 50mg cimetidine, and
333.3 mg of uncoated matrix pellets comprising 250mg cimetidine.

### Example 3

Uncoated immediate-release cimetidine pellets, coated matrix pellets, and immediate coated pellets are contained within a hard gelatin capsule.

The immediate coated cimetidine pellets and immediate release pellets are prepared as in Example 1, above.

### Coating of the Uncoated Polymer Matrix Pellets

### (Coated Matrix Pellets)

The uncoated matrix cimetidine pellet is first prepared using the procedures described above in Example 1 for preparation of the un-coated Cimetidine Polymer Matrix pellets. The uncoated pellet is then coated in an analagous manner and with the same coating suspension as described in Example 1 for the immediate coated pellets. When prepared in accordance with these procedures the coated pellets contain 75/120 = 62.5% of cimetidine.

### Encapsulation

The coated and uncoated pellets as prepared above are filled (encapsulated) into capsules, such that one capsule contains:
58.8 mg of uncoated immediate release pellets comprising 50 mg cimetidine;
141.2 mg of immediate coated pellets comprising 100mg cimetidine; and
240.0 mg of coated matrix pellets comprising 150 mg cimitidine.

Thus two capsules provides a 600mg dose of cimetidine wherein the ratio of immediate release, immediate coated and coated matrix is 1:2:3 [50+100+150 = 300mg].

### Example 4

Uncoated immediate-release cimetidine pellets, and coated matrix pellets are contained within a hard gelatin capsule.

The immediate release pellets are prepared as described above, in Example 1. The coated matrix pellets are prepared as described in Example 3. These pellets are filled (encapsulated) into capsules, such that one capsule contains:
117.6mg of uncoated immediate release pellets comprising 100mg cimetidine, and
320mg of coated matrix pellets comprising 200mg cimetidine.

Alternatively, the pellets may be encapsulated such that one capsule contains:
88.2 mg of uncoated pellets comprising 75mg cimetidine, and
360.0 mg of coated matrix pellets comprising 225mg cimetidine.

### Example 5

Uncoated immediate-release cimetidine pellets, uncoated matrix pellets, coated matrix pellets, and immediate coated pellets are contained within a hard gelatin capsule.

### Manufacture:

The uncoated matrix is prepared as described above in Example 1. The coated matrix is prepared, as described above, in Example 3. The immediate release and immediate coated release pellets are also prepared, as described above, in Example 1.

### Encapsulation

Uncoated and coated pellets are filled into capsules such that one capsule contains:
29.4 mg of uncoated pellets comprising 25 mg cimetidine (8.3%)
35.3 mg of coated pellets (immediate-coated) comprising 25 mg cimetidine (8.3%)
133.3 mg of uncoated polymer matrix-pellets comprising 100 mg cimetidine (33.3%)
240.0 mg of coated polymer matrix-pellets comprising 150 mg cimetidine (50%).

The above description fully discloses the invention including preferred embodiments thereof. The embodiments of the invention in which an exclusive property or privilege is claimed are defined as follows.

## Claims (Claims for the following Contracting State(s): AT, BE, CH, DE, DK, FR, GB, IT, LI, LU, MC, NL, SE)

1. A pharmaceutical composition comprising an H₂ antagonist characterised in that the H₂ antagonist is present in a modified-release phase comprising a polymer matrix which is selected from the group consisting of a non-ionic neutral copolymer of ethyl acrylate and methyl acrylate, acrylic and methacrylic acid esters, ethyl cellulose, hydroxypropyl methylcellulose, gelatin, waxes or mixtures thereof; the polymer matrix material being present in an amount of 10 to 20% (w/w) of polymer relative to H₂ antagonist.

2. The pharmaceutical composition according to claim 1 wherein the H₂ antagonist is cimetidine.

3. The pharmaceutical composition according to claim 1 or 2 which comprises a plurality of polymer matrix cores.

4. The pharmaceutical composition according to any one of claims 1 to 3 wherein the polymer matrix is a co-polymer of Eudragit™RL 30D.

5. The pharmaceutical composition according to any one of claims 1 to 4 wherein the modified-release phase is composed of pellets.

6. The pharmaceutical composition according to claim 5 wherein the pellets are contained in a gelatin capsule.

7. The pharmaceutical composition according to claim 3 wherein at least one of the matrixes is independently coated with a release-delaying substance which is selected from the group consisting of co-polymers based on polymethacrylic acid and methacrylates, ethyl acrylate and methyl acrylate, co-polymers of acrylic and methacrylic acid esters, co-polymers of Eudragit™RL 30D, hydroxypropyl methylcellulose phthlate, cellulose acetate phthalate, polyvinyl acetate pthtalate or mixtures thereof; and is present in an amount of from 2 to 30% (w/w) relative to the matrix-core.

8. The pharmaceutical composition according to claim 7 wherein the coating agent is a copolymer of polymethacrylic acid and methacrylates, methacrylic acid and ethylacrylate, or copolymers of Eudragit™RL 30D.

9. The pharmaceutical composition according to any one of claims 1 to 8 which further comprises an immediate release phase wherein the modified-release phase is present in an amount of 66 to 75% (w/w) relative to the immediate release phase.

10. The pharmaceutical composition according to claim 9 wherein the matrix core is optionally coated with a release-delaying substance, and wherein the immediate release phase is optionally coated with a release-delaying substance.

11. A pharmaceutical composition according to claim 9 or 10 comprising:
a) an immediate-release phase of cimetidine;
b) an immediate coated release phase of cimetidine which comprises at least one core of cimetidine coated with a release-delaying substance in an amount of from 2 to 30% (w/w) relative to the core;
c) a modified-release phase which comprises cimetidine incorporated into a polymer matrix optionally coated with a release-delaying substance in an amount of from 2 to 30% (w/w) relative to the matrix-core.

12. The pharmaceutical composition according to claim 11 wherein the modified release phase is present in an amount of 66 to 75% (w/w) relative to the immediate release phase.

13. The pharmaceutical composition according to claim 11 or 12 wherein the polymer matrix material is present in an amount of 10 to 20% (w/w) of polymer relative to cimetidine.

14. A pharmaceutical composition according to claim 1 comprising:
a) an immediate release phase of cimetidine;
b) a modified-release phase which comprises cimetidine incorporated into a polymer matrix;
c) a second modified-release phase which comprises cimetidine incorporated into a polymer matrix coated with a release-delaying substance in an amount of from 2 to 30% (w/w) relative to the matrix-core; and
d) a third modified-release phase which comprises cimetidine coated with a release-delaying substance in an amount of from 2 to 30% (w/w) relative to the core.

## Claims (Claims for the following Contracting State(s): ES, GR)

1. A pharmaceutical composition comprising an H₂ antagonist characterised in that the H₂ antagonist is present in a modified-release phase comprising a polymer matrix which is selected from the group consisting of a non-ionic neutral copolymer of ethyl acrylate and methyl acrylate, acrylic and methacrylic acid esters, ethyl cellulose, hydroxypropyl methylcellulose, gelatin, waxes or mixtures thereof; the polymer matrix material being present in an amount of 10 to 20% (w/w) of polymer relative to H₂ antagonist.

2. The pharmaceutical composition according to claim 1 wherein the H₂ antagonist is cimetidine.

3. The pharmaceutical composition according to claim 1 or 2 which comprises a plurality of polymer matrix cores.

4. The pharmaceutical composition according to any one of claims 1 to 3 wherein the polymer matrix is a co-polymer of Eudragit™RL 30D.

5. The pharmaceutical composition according to any one of claims 1 to 4 wherein the modified-release phase is composed of pellets.

6. The pharmaceutical composition according to claim 5 wherein the pellets are contained in a gelatin capsule.

7. The pharmaceutical composition according to claim 3 wherein at least one of the matrixes is independently coated with a release-delaying substance which is selected from the group consisting of co-polymers based on polymethacrylic acid and methacrylates, ethyl acrylate and methyl acrylate, co-polymers of acrylic and methacrylic acid esters, co-polymers of Eudragit™RL 30D, hydroxypropyl methylcellulose phthlate, cellulose acetate phthalate, polyvinyl acetate pthtalate or mixtures thereof; and is present in an amount of from 2 to 30% (w/w) relative to the matrix-core.

8. The pharmaceutical composition according to claim 7 wherein the coating agent is a copolymer of polymethacrylic acid and methacrylates, methacrylic acid and ethylacrylate, or copolymers of Eudragit™RL 30D.

9. The pharmaceutical composition according to any one of claims 1 to 8 which further comprises an immediate release phase wherein the modified-release phase is present in an amount of 66 to 75% (w/w) relative to the immediate release phase.

10. The pharmaceutical composition according to claim 9 wherein the matrix core is optionally coated with a release-delaying substance, and wherein the immediate release phase is optionally coated with a release-delaying substance.

11. A pharmaceutical composition according to claim 9 or 10 comprising:
a) an immediate release phase of cimetidine;
b) an immediate coated release phase of cimetidine which comprises at least one core of cimetidine coated with a release-delaying substance in an amount of from 2 to 30% (w/w) relative to the core;
c) a modified-release phase which comprises cimetidine incorporated into a polymer matrix optionally coated with a release-delaying substance in an amount of from 2 to 30% (w/w) relative to the matrix-core.

12. The pharmaceutical composition according to claim 11 wherein the modified release phase is present in an amount of 66 to 75% (w/w) relative to the immediate release phase.

13. The pharmaceutical composition according to claim 11 or 12 wherein the polymer matrix material is present in an amount of 10 to 20% (w/w) of polymer relative to cimetidine.

14. A pharmaceutical composition according to claim 1 comprising:
a) an immediate-release phase of cimetidine;
b) a modified-release phase which comprises cimetidine incorporated into a polymer matrix;
c) a second modified-release phase which comprises cimetidine incorporated into a polymer matrix coated with a release-delaying substance in an amount of from 2 to 30% (w/w) relative to the matrix-core; and
d) a third modified-release phase which comprises cimetidine coated with a release-delaying substance in an amount of from 2 to 30% (w/w) relative to the core.

15. A process for the preparation of a pharmaceutical composition comprising an H₂ antagonist in a modified-release phase which process comprises granulating the H₂ antagonist in a polymer matrix which is selected from the group consisiting of a non-ionic neutral copolymer of ethyl acrylate and methyl acrylate, acrylic and methacrylic acid esters, ethyl cellulose, hydroxypropyl methylcellulose, gelatin, waxes or mixtures thereof; and the polymer matrix material is present in an amount of 10 to 20% (w/w) of polymer relative to H₂ antagonist.

## Patentansprüche (Patentansprüche für folgende(n) Vertragsstaat(en): AT, BE, CH, DE, DK, FR, GB, IT, LI, LU, MC, NL, SE)

1. Arzneimittel, umfassend einem H₂-Antagonisten, dadurch gekennzeichnet, daß der H₂-Antagonist in einer Phase für eine modifizierte Freisetzung vorliegt, die eine Polymermatrix umfaßt, die ausgewählt ist aus einem nichtionischen neutralen Copolymer aus Ethylacrylat und Methylacrylat, Acryl- und Methacrylsäureestern, Ethylcellulose, Hydroxypropylmethylcellulose, Gelatine, Wachsen oder Gemischen hiervon; wobei die Polymermatrix in einer Menge von 10 bis 20 % (Gew./Gew.) des Polymers bezogen auf den H₂-Antagonisten vorliegt.

2. Arzneimittel nach Anspruch 1, wobei der H₂-Antagonist Cimetidin ist.

3. Arzneimittel nach Anspruch 1 oder 2, das eine Vielzahl von Polymermatrixkernen umfaßt.

4. Arzneimittel nach einem der Ansprüche 1 bis 3, wobei die Polymermatrix ein Copolymer von Eudragit™RL 30D ist.

5. Arzneimittel nach einem der Ansprüche 1 bis 4, wobei die Phase für eine modifizierte Freisetzung aus Kügelchen besteht.

6. Arzneimittel nach Anspruch 5, wobei sich die Kügelchen in einer Gelatinekapsel befinden.

7. Arzneimittel nach Anspruch 3, wobei mindestens eine der Matrizen unabhängig mit einem die Freisetzung verzögernden Stoff überzogen ist, ausgewählt aus Copolymeren, basierend auf Polymethacrylsäure und Methacrylaten, Ethylacrylat und Methylacrylat, Copolymeren aus Acryl- und Methacrylsäureestern, Copolymeren auf Eudragit™RL 30D, Hydroxypropylmethylcellulosephthalat, Celluloseacetatphthalat, Polyvinylacetatphthalat oder Gemische hiervon; und in einer Menge von 2 bis 30 % (Gew./Gew.) bezogen auf den Matrixkern vorliegt.

8. Arzneimittel nach Anspruch 7, wobei das Überzugsmittel ein Copolymer aus Polymethacrylsäure und Methacrylaten, Methacrylsäure und Ethylacrylat oder Copolymere von Eudragit™RL 30D ist.

9. Arzneimittel nach einem der Ansprüche 1 bis 8, das weiterhin eine Phase für die unmittelbare Freisetzung umfaßt, wobei die Phase für eine modifizierte Freisetzung in einer Menge von 66 bis 75 % (Gew./Gew.) bezogen auf die Phase für die unmittelbare Freisetzung vorliegt.

10. Arzneimittel nach Anspruch 9, wobei dar Matrixkern gegebenenfalls überzogen ist mit einem die Freisetzung verzögernden Stoff und wobei die Phase für die unmittelbare Freisetzung gegebenenfalls überzogen ist mit einem die Freisetzung verzögernden Stoff.

11. Arzneimittel nach Anspruch 9 oder 10, umfassend:
a) eine Phase für die unmittelbare Freisetzung aus Cimetidin;
b) eine überzogene Phase für die unmittelbare Freisetzung aus Cimetidin, welche mindestens einen Kern von Cimetidin umfaßt, der überzogen ist mit einem die Freisetzung verzögernden Stoff in einer Menge von 2 bis 33 % (Gew./Gew.) bezogen auf den Kern,
c) eine Phase für eine modifizierte Freisetzung, die Cimetidin, das in eine Polymermatrix aufgenommen ist, umfaßt, gegebenenfalls überzogen mit einem die Freisetzung verzögernden Stoff in einer Menge von 2 bis 30 % (Gew./Gew.) bezogen auf den Matrixkern.

12. Arzneimittel nach Anspruch 11, wobei die Phase für eine modifizierte Freisetzung in einer Menge von 66 bis 75 % (Gew./Gew.) im Verhältnis zur Phase für die unmittelbaren Freisetzung vorliegt.

13. Arzneimittel nach Anspruch 11 oder 12, wobei das Polymermatrixmaterial in einer Menge von 10 bis 20 % (Gew./Gew.) des Polymers bezogen auf Cimetidin vorliegt.

14. Arzneimittel nach Anspruch 1, umfassend:
a) eine Phase für die unmittelbare Freisetzung aus Cimetidin,
b) eine Phase für eine modifizierte Freisetzung, die Cimetidin umfaßt, das in eine Polymermatrix aufgenommen ist;
c) eine zweite Phase für eine modifizierte Freisetzung, welche Cimetidin umfaßt, das in einer Polymermatrix aufgenommen ist, die überzogen ist mit einem die Freisetzung verzögernden Stoff in einer Menge von 2 bis 30 % (Gew./Gew.) bezogen auf den Matrixkern; und
d) eine dritte Phase für eine modifizierte Freisetzung, die Cimetidin umfaßt, welche überzogen ist mit einem die Freisetzung verzögernden Stoff in einer Menge von 2 bis 30 % (Gew./Gew.) bezogen auf den Kern.

## Patentansprüche (Patentansprüche für folgende(n) Vertragsstaat(en): ES, GR)

1. Arzneimittel, umfassend einen H₂-Antagonisten, dadurch gekennzeichnet, daß der H₂-Antagonist in einer Phase für eine modifizierte Freisetzung vorliegt, die eine Polymermatrix umfaßt, die ausgewählt ist aus einem nichtionischen neutralen Copolymer aus Ethylacrylat und Methylacrylat, Acryl- und Methacrylsäureestern, Ethylcellulose, Hydroxypropylmethylcellulose, Gelatine, Wachsen oder Gemischen hiervon; wobei die Polymermatrix in einer Menge von 10 bis 20 % (Gew./Gew.) des Polymers bezogen auf den H₂-Antagonisten vorliegt.

2. Arzneimittel nach Anspruch 1, wobei der H₂-Antagonist Cimetidin ist.

3. Arzneimittel nach Anspruch 1 oder 2, das eine Vielzahl von Polymermatrixkernen umfaßt.

4. Arzneimittel nach einem der Ansprüche 1 bis 3, wobei die Polymermatrix ein Copolymer von Eudragit™RL 30D ist.

5. Arzneimittel nach einem der Ansprüche 1 bis 4, wobei die Phase für eine modifizierte Freisetzung aus Kügelchen besteht.

6. Arzneimittel nach Anspruch 5, wobei sich die Kügelchen in einer Gelatinekapsel befinden.

7. Arzneimittel nach Anspruch 3, wobei mindestens eine der Matrizen unabhängig mit einen die Freisetzung verzögernden Stoff überzogen ist, ausgewählt aus Copolymeren, basierend auf Polymethacrylsäure und Methacrylaten, Ethylacrylat und Methylacrylat, Copolymeren aus Acryl- und Methacrylsäureestern, Copolymeren auf Eudragit™RL 30D, Hydroxypropylmethylcellulosephthalat, Celluloseacetatphthalat, Polyvinylacetatphthalat oder Gemische hiervon; und in einer Menge von 2 bis 30 % (Gew./Gew.) bezogen auf den Matrixkern vorliegt.

8. Arzneimittel nach Anspruch 7, wobei das Überzugsmittel ein Copolymer aus Polymethacrylsäure und Methacrylaten, Methacrylsäure und Ethylacrylat oder Copolymere von Eudragit™RL 30D ist.

9. Arzneimittel nach einem der Ansprüche 1 bis 8, das weiterhin eine Phase für die unmittelbare Freisetzung umfaßt, wobei die Phase für eine modifizierte Freisetzung in einer Menge von 66 nie 75 % (Gew./Gew.) bezogen auf die Phase für die urmittelbare Freisetzung vorliegt.

10. Arzneimittel nach Anspruch 9, wobei der Matrixkern gegebenenfalls überzogen ist mit einem die Freisetzung verzögernden Stoff und wobei die Phase für die unmittelbare Freisetzung gegebenenfalls überzogen ist mit einem die Freisetzung verzögernden Stoff.

11. Arzneimittel nach Anspruch 9 oder 10, umfassend:
a) eine Phase für die unmittelbare Freisetzung aus Cimetidin;
b) eine überzogene Phase für die unmittelbare Freisetzung aus Cimetidin, welche mindestens einen Kern von Cimetidin umfaßt, der überzogen ist mit einem die Freisetzung verzögernden Stoff in einer Menge von 2 bis 30 % (Gew./Gew.) bezogen auf den Kern,
c) eine Phase für eine modifizierte Freisetzung, die Cimetidin, das in eine Polymermatrix aufgenommen ist, umfaßt, gegebenenfalls überzogen mit einem die Freisetzung verzögernden Stoff in einer Menge von 2 bis 30 % (Gew./Gew.) bezogen auf den Matrixkern.

12. Arzneimittel nach Anspruch 11, wobei die Phase für eine modifizierte Freisetzung in einer Menge von 66 bis 75 % (Gew./Gew.) im Verhältnis zur Phase für die unmittelbaren Freisetzung vorliegt.

13. Arzneimittel nach Anspruch 11 oder 12, wobei das Polymermatrixmaterial in einer Menge von 10 bis 20 % (Gew./Gew.) des Polymers bezogen auf Cimetidin vorliegt.

14. Arzneimittel nach Anspruch 1, umfassend:
a) eine Phase für die unmittelbare Freisetzung aus Cimetidin;
b) eine Phase für eine modifizierte Freisetzung, die Cimetidin umfaßt, das in eine Polymermatrix aufgenommen ist;
c) eine zweite Phase für eine modifizierte Freisetzung, welche Cimetidin umfaßt, das in einer Polymermatrix aufgenommen ist, die überzogen ist mit einem die Freisetzung verzögernden Stoff in einer Menge von 2 bis 30 % (Gew./Gew.) bezogen auf den Matrixkern, und
d) eine dritte Phase für eine modifizierte Freisetzung, die Cimetidin umfaßt, welche überzogen ist mit einem die Freisetzung verzögernden Stoff in einer Menge von 2 bis 30 % (Gew./Ges.) bezogen auf den Kern.

15. Verfahren zur Herstellung eines Arzneimittels, umfassend einen H₂-Antagonisten in einer Phase für eine modifizierte Freisetzung, wobei das Verfahren umfaßt: das Granulieren des H₂-Antagonisten in einer Polymermatrix, die ausgewählt ist aus einem nicht-ionischen neutralen Copolymer aus Ethylacrylat und Methylacrylat, Acryl- und Methacrylsäureestern, Ethylcellulose, Hydroxypropylmethylcellulose, Gelatine, Wachsen oder Gemischen hiervon; und das Polymermatrixmaterial in einer Menge von 10 bis 20 % (Gew./Gew.) des Polymers bezogen auf den H₂-Antagonisten vorliegt.

## Revendications (Revendications pour l'(les) Etat(s) contractant(s) suivant(s): AT, BE, CH, DE, DK, FR, GB, IT, LI, LU, MC NL, SE)

1. Composition pharmaceutique comprenant un antagoniste H₂, caractérisée en ce que l'antagoniste H₂ est présent dans une phase à libération modifiée comprenant une matrice polymérique qui est choisie dans le groupe consistant en un copolymère neutre non ionique d'acrylate d'éthyle et d'acrylate de méthyle, des esters d'acide acrylique et d'acide méthacrylique, l'éthylcellulose, l'hydroxypropylméthylcellulose, la gélatine, des cires et leurs mélanges ; la matière de matrice polymérique étant présente en une quantité de 10 à 20 % (en poids/poids) de polymère par rapport à l'antagoniste H₂.

2. Composition pharmaceutique suivant la revendication 1, dans laquelle l'antagoniste H₂ est la cimétidine.

3. Composition pharmaceutique suivant la revendication 1 ou 2, qui comprend une pluralité de noyaux de matrice polymérique.

4. Composition pharmaceutique suivant l'une quelconque des revendications 1 à 3, dans laquelle la matrice polymérique est constituée d'un copolymère de Eudragit™RL 30D.

5. Composition pharmaceutique suivant l'une quelconque des revendications 1 à 4, dans laquelle la phase à libération modifiée est constituée de granules.

6. Composition pharmaceutique suivant la revendication 5, dans laquelle les granules sont présents dans une capsule de gélatine.

7. Composition pharmaceutique suivant la revendication 3, dans laquelle au moins une des matrices est enrobée, indépendamment, avec une substance retardant la libération qui est choisie dans le groupe consistant en des copolymères à base d'acide polyméthacrylique et de méthacrylates, d'acrylate d'éthyle et d'acrylate de méthyle, des copolymères d'esters d'acide acrylique et d'acide méthacrylique, des copolymères de Eudragit™RL 30D, le phtalate d'hydroxypropylméthylcellulose, l'acétophtalatedecellulose, l'acétophtalate de polyvinyle et leurs mélanges ; et est présente en une quantité de 2 à 30 % (en poids/poids) par rapport au noyau-matrice.

8. Composition pharmaceutique selon la revendication 7, dans laquelle l'agent d'enrobage est un copolymère d'acide polyméthacrylique et de méthacrylates, d'acide méthacrylique et d'acrylate d'éthyle, ou un copolymère de Eudragit™RL 30D.

9. Composition pharmaceutique suivant l'une quelconque des revendications 1 à 8, qui comprend en outre une phase à libération immédiate, dans laquelle la phase à libération modifiée est présente en une quantité de 66 à 75 % (en poids/poids) par rapport à la phase à libération immédiate.

10. Composition pharmaceutique suivant la revendication 9, dans laquelle le noyau-matrice est facultativement enrobé avec une substance retardant la libération, et dans laquelle la phase à libération immédiate est facultativement enrobée avec une substance retardant la libération.

11. Composition pharmaceutique suivant la revendication 9 ou 10, comprenant :
a) une phase à libération immédiate de cimétidine ;
b) une phase à libération immédiate de cimétidine, enrobée, qui comprend au moins un noyau de cimétidine enrobé avec une substance retardant la libération en une quantité de 2 à 30 % (en poids/poids) par rapport au noyau ;
c) une phase à libération modifiée qui comprend de la cimétidine incorporée à une matrice polymérique facultativement enrobée avec une substance retardant la libération en une quantité de 2 à 30 % (en poids/poids) par rapport au noyau-matrice.

12. Composition pharmaceutique suivant la revendication 11, dans laquelle la phase à libération modifiée est présente en une quantité de 66 à 75 % (en poids/poids) par rapport à la phase à libération immédiate.

13. Composition pharmaceutique suivant la revendication 11 ou 12, dans laquelle la matière de matrice polymérique est présente en une quantité de 10 à 20 % (en poids/poids) de polymère par rapport à la cimétidine.

14. Composition pharmaceutique suivant la revendication 1, comprenant :
a) une phase à libération immédiate de cimétidine ;
b) une phase à libération modifiée, qui comprend de la cimétidine incorporée à une matrice polymérique ;
c) une deuxième phase à libération modifiée, qui comprend la cimétidine incorporée à une matrice polymérique enrobée avec une substance retardant la libération en une quantité de 2 à 30 % (en poids/poids) par rapport au noyau-matrice ; et
d) une troisième phase à libération modifiée qui comprend la cimétidine enrobée avec une substance retardant la libération en une quantité de 2 à 30 % (en poids/poids) par rapport au noyau.

## Revendications (Revendications pour l'(les) Etat(s) contractant(s) suivant(s): ES, GR)

1. Composition pharmaceutique comprenant un antagoniste H₂, caractérisée en ce que l'antagoniste H₂ est présent dans une phase à libération modifiée comprenant une matrice polymérique qui est choisie dans le groupe consistant en un copolymère neutre non ionique d'acrylate d'éthyle et d'acrylate de méthyle, des esters d'acide acrylique et d'acide méthacrylique, l'éthylcellulose, l'hydroxypropylméthylcellulose, la gélatine, des cires et leurs mélanges ; la matière de matrice polymérique étant présente en une quantité de 10 à 20 % (en poids/poids) de polymère par rapport à l'antagoniste H₂.

2. Composition pharmaceutique suivant la revendication 1, dans laquelle l'antagoniste H₂ est la cimétidine.

3. Composition pharmaceutique suivant la revendication 1 ou 2, qui comprend une pluralité de noyaux de matrice polymérique.

4. Composition pharmaceutique suivant l'une quelconque des revendications 1 à 3, dans laquelle la matrice polymérique est constituée d'un copolymère de Eudragit™RL 30D.

5. Composition pharmaceutique suivant l'une quelconque des revendications 1 à 4, dans laquelle la phase à libération modifiée est constituée de granules.

6. Composition pharmaceutique suivant la revendication 5, dans laquelle les granules sont présents dans une capsule de gélatine.

7. Composition pharmaceutique suivant la revendication 3, dans laquelle au moins une des matrices est enrobée, indépendamment, avec une substance retardant la libération qui est choisie dans le groupe consistant en des copolymères à base d'acide polyméthacrylique et de méthacrylates, d'acrylate d'éthyle et d'acrylate de méthyle, des copolymères d'esters d'acide acrylique et d'acide méthacrylique, des copolymères de Eudragit™RL 30D, le phtalate d'hydroxypropylméthylcellulose, l'acétophtalate de cellulose, l'acétophtalate de polyvinyle et leurs mélanges ; et est présente en une quantité de 2 à 30 % (en poids/poids) par rapport au noyau-matrice.

8. Composition pharmaceutique selon la revendication 7, dans laquelle l'agent d'enrobage est un copolymère d'acide polyméthacrylique et de méthacrylates, d'acide méthacrylique et d'acrylate d'éthyle, ou un copolymère de Eudragit™RL 30D.

9. Composition pharmaceutique suivant l'une quelconque des revendications 1 à 8, qui comprend en outre une phase à libération immédiate, dans laquelle la phase à libération modifiée est présente en une quantité de 66 à 75 % (en poids/poids) par rapport à la phase à libération immédiate.

10. Composition pharmaceutique suivant la revendication 9, dans laquelle le noyau-matrice est facultativement enrobé avec une substance retardant la libération, et dans laquelle la phase à libération immédiate est facultativement enrobée avec une substance retardant la libération.

11. Composition pharmaceutique suivant la revendication 9 ou 10, comprenant :
a) une phase à libération immédiate de cimétidine ;
b) une phase à libération immédiate de cimétidine, enrobée, qui comprend au moins un noyau de cimétidine enrobé avec une substance retardant la libération en une quantité de 2 à 30 % (en poids/poids) par rapport au noyau ;
c) une phase à libération modifiée, qui comprend de la cimétidine incorporée à une matrice polymérique facultativement enrobée avec une substance retardant la libération en une quantité de 2 à 30 % (en poids/poids) par rapport au noyau-matrice.

12. Composition pharmaceutique suivant la revendication 11, dans laquelle la phase à libération modifiée est présente en une quantité de 66 à 75 % (en poids/poids) par rapport à la phase à libération immédiate.

13. Composition pharmaceutique suivant la revendication 11 ou 12, dans laquelle la matière de matrice polymérique est présente en une quantité de 10 à 20 % (en poids/poids) de polymère par rapport à la cimétidine.

14. Composition pharmaceutique suivant la revendication 1, comprenant :
a) une phase à libération immédiate de cimétidine ;
b) une phase à libération modifiée, qui comprend de la cimétidine incorporée à une matrice polymérique ;
c) une deuxième phase à libération modifiée, qui comprend de la cimétidine incorporée à une matrice polymérique enrobée avec une substance retardant la libération en une quantité de 2 à 30 % (en poids/poids) par rapport au noyau-matrice ; et
d) une troisième phase à libération modifiée, qui comprend de la cimétidine enrobée avec une substance retardant la libération en une quantité de 2 à 30 % (en poids/poids) par rapport au noyau.

15. Procédé pour la préparation d'une composition pharmaceutique comprenant un antagoniste H₂ dans une phase à libération modifiée, procédé qui comprend la granulation de l'antagoniste H₂ dans une matrice polymérique qui est choisie dans le groupe consistant en un copolymère neutre non ionique d'acrylate d'éthyle et d'acrylate de méthyle, des esters d'acide acrylique et d'acide méthacrylique, l'éthylcellulose, l'hydroxypropylméthylcellulose, la gélatine, des cires et leurs mélanges ; la matière de matrice polymérique étant présente en une quantité de 10 à 20 % (en poids/poids) de polymère par rapport à l'antagoniste H₂.
